# EUROPEAN PATENT SPECIFICATION

(11) **EP 3 679 787 B1**
(45) Date of publication and mention of the grant of the patent: **01.03.2023**
(21) Application number: 19191092.6
(22) Date of filing: 09.08.2019
(51) Int. Cl.: A01K 29/00

(54) **METHOD AND SYSTEM FOR MONITORING EPIDEMIC DISEASE IN PIGGERY**
VERFAHREN UND SYSTEM ZUR ÜBERWACHUNG VON EPIDEMISCHEN ERKRANKUNGEN IN DER SCHWEINEZUCHT
PROCÉDÉ ET SYSTÈME DE SURVEILLANCE D'ÉPIDÉMIES DANS UNE PORCHERIE

(30) Priority: 08.01.2019 CN 201910015002
(43) Date of publication of application: 15.07.2020
(73) Proprietor: Beijing Etag Technology Company., Ltd., Haidian District Beijing (CN)
(72) Inventor: ZHOU, Xingfu, Beijing (CN); REN, Jiping, Beijing (CN); PANG, Chao, Beijing (CN); HU, Dongge, Beijing (CN)
(74) Representative: EP&C

(56) References cited:
- WO-A1-02/060244
- CN-A- 108 037 717
- GB-A- 2 437 250
- TW-A- 201 227 548
- WARREN S ET AL: "A distributed infrastructure for veterinary telemedicine", PROCEEDINGS OF THE 25TH. ANNUAL INTERNATIONAL CONFERENCE OF THE IEEE ENGINEERING IN MEDICINE AND BIOLOGY SOCIETY. CANCUN, MEXICO, SEPT. 17 - 21, 2003; [ANNUAL INTERNATIONAL CONFERENCE OF THE IEEE ENGINEERING IN MEDICINE AND BIOLOGY SOCIETY. EMBS], NE, vol. 2, 17 September 2003 (2003-09-17), pages 1394-1397, XP010693138, DOI: 10.1109/IEMBS.2003.1279573 ISBN: 978-0-7803-7789-9

## Description

### TECHNICAL FIELD

The present invention relates to the field of monitoring of epidemic diseases in a piggery, and in particular to a method and system for monitoring an epidemic disease in a piggery.

### BACKGROUND

Epidemic diseases in a piggery have a huge impact, but there hasn't been an effective solution to provide a breeding guarantee for a farmer till now and the early detection and early treatment cannot be achieved. Existing epidemic diseases in a piggery are monitored artificially. However, due to the fast spreading of the epidemic diseases in a piggery, it is too late for the detection of the farmer, thus causing an enormous loss for the farmer or even affecting an order of a nationwide economic market.

CN 108037717 A discloses a smart pig farm monitoring system. The monitoring system includes a water supply device, a food supply device and a processing module; the water supply device includes a plurality of water supply units, wherein the water supply units are respectively arranged in pigsties in a pig farm and are used for collecting the drinking water data of pigs to be tested, the identity information of the pigs to be tested, and pigsty identifiers; the food supply device includes a plurality of food supply units, wherein the food supply units are respectively arranged in the pigsties in the pig farm and are used for collecting the dietary data of the pigs to be tested, the identity information of the pigs to be tested, and the pig identifiers; and the processing module is connected with the water supply device and the food supply device and is used for obtaining pre-stored bred pig reference data according to the pigsty identifiers, comparing the drinking water data of the pigs to be tested and the dietary data of the pigs to be tested with the drinking water reference value of the corresponding pigs to be tested and the dietary reference value of the corresponding pigs to be tested in the bred pig reference data respectively, and outputting the health statuses of the pigs to be tested according to comparison results.

### SUMMARY

To achieve the above objective, the present invention provides the following solution.

A method for monitoring an epidemic disease in a piggery includes:
obtaining a historical food intake amount, historical environmental parameters, a historical health state and a historical epidemic disease state of a live pig, where the historical environmental parameters include a temperature, a humidity, and concentration of gas in the air;
establishing a piggery monitoring model by taking the historical food intake amount and the historical environmental parameters as an input and the historical health state and the historical epidemic disease state as an output;
determining a first current state of the live pig according to the piggery monitoring model, where the first current state includes a healthy state and an epidemic disease state; and
monitoring a monitored piggery according to the first current state, wherein determining the first current state of the live pig according to the piggery monitoring model specifically includes:
   obtaining a current food intake amount of the live pig within a period of time threshold range;
   determining whether the current food intake amount is in a downtrend within the time threshold range to obtain a first determining result;
   if the first determining result indicates that the current food intake amount is in the downtrend within the time threshold range, obtaining current environmental parameters of the monitored piggery;
   determining whether the current environmental parameters and the historical environmental parameters are within an environmental-parameter similarity threshold range to obtain a second determining result; and
   if the second determining result indicates that the current environmental parameters and the historical environmental parameters are within the environmental-parameter similarity threshold range, determining, by taking the current food intake amount and the current environmental parameters as an input, the first current state of the live pig according to the piggery monitoring model.

Optionally, after monitoring the monitored piggery according to the first current state, the method further includes:
obtaining a second current state of any live pig in the monitored piggery;
determining whether the second current state and the first current state are within a first current-state similarity threshold range to obtain a third determining result;
if the third determining result indicates that the second current state and the first current state are within the first current-state similarity threshold range, determining that no epidemic disease occurs in the monitored piggery; and
if the third determining result indicates that the second current state and the first current state are not within the first current-state similarity threshold range, determining that an epidemic disease occurs in the monitored piggery and making an alarm to a farmer.

Optionally, after determining that an epidemic disease occurs in the monitored piggery and making the alarm to the farmer, the method further includes:
obtaining a third current state of a live pig in any piggery;
determining whether the third current state and the first current state are within a second current-state similarity threshold range to obtain a fourth determining result;
if the third current state and the first current state are within the second current-state similarity threshold range, determining that the epidemic disease does not occur in the any piggery; and
if the third current state and the first current state are not within the second current-state similarity threshold range, determining that the epidemic disease occurs in the any piggery and making an alarm to the farmer.

A system for monitoring an epidemic disease in a piggery includes:
a parameter obtaining module, configured to obtain a historical food intake amount, historical environmental parameters, a historical health state and a historical epidemic disease state of a live pig, where the historical environmental parameters include a temperature, a humidity, and concentration of gas in the air;
a piggery monitoring model establishment module, configured to establish a piggery monitoring model by taking the historical food intake amount and the historical environmental parameters as an input and the historical health state and the historical epidemic disease state as an output;
a first current state determination module, configured to determine a first current state of the live pig according to the piggery monitoring model, where the first current state includes a healthy state and an epidemic disease state; and
a monitoring module, configured to monitor a monitored piggery according to the first current state, wherein
the first current state determination module specifically includes:
   a current food intake amount obtaining unit, configured to obtain a current food intake amount of the live pig within a period of time threshold range;
   a first judgment unit, configured to determine whether the current food intake amount is in a downtrend within the time threshold range to obtain a first determining result;
   a current environmental parameter obtaining unit, configured to obtain, if the first determining result indicates that the current food intake amount is in the downtrend within the time threshold range, current environmental parameters of the monitored piggery;
   a second judgment unit, configured to determine whether the current environmental parameters and the historical environmental parameters are within an environmental-parameter similarity threshold range to obtain a second determining result; and
   a first current state determination unit, configured to determine, if the second determining result indicates that the current environmental parameters and the historical environmental parameters are within the environmental-parameter similarity threshold range, by taking the current food intake amount and the current environmental parameters as an input, the first current state of the live pig according to the piggery monitoring model.

Optionally, the system for monitoring an epidemic disease in a piggery further includes:
a second current state obtaining module, configured to obtain a second current state of any live pig in the monitored piggery;
a third determining module, configured to determine whether the second current state and the first current state are within a first current-state similarity threshold range to obtain a third determining result;
a monitored piggery monitoring module, configured to determine, if the third determining result indicates that the second current state and the first current state are within the first current-state similarity threshold range, that no epidemic disease occurs in the monitored piggery; and
a first alarm module, configured to determine, if the third determining result indicates that the second current state and the first current state are not within the first current-state similarity threshold range, that an epidemic disease occurs in the monitored piggery and make an alarm to a farmer.

Optionally, the system for monitoring an epidemic disease in a piggery further includes:
a third current state obtaining module, configured to obtain a third current state of a live pig in any piggery;
a fourth determining module, configured to determine whether the third current state and the first current state are within a second current-state similarity threshold range to obtain a fourth determining result;
an any piggery monitoring module, configured to determine, if the third current state and the first current state are within the second current-state similarity threshold range, that the epidemic disease does not occur in the any piggery; and
a second alarm module, configured to determine, if the third current state and the first current state are not within the second current-state similarity threshold range, that the epidemic disease occurs in the any piggery and make an alarm to the farmer.

According to the specific embodiments provided by the present invention, the present invention discloses the following technical effects: with the adoption of the method for monitoring an epidemic disease in a piggery and system provided by the present invention, a piggery monitoring model is established, by taking the historical food intake amount and the historical environmental parameters as an input and the historical health state and the historical epidemic disease state as an output, according to a historical food intake amount, historical environmental parameters, a historical health state and a historical epidemic disease state of a live pig; and a current state of the live pig in a monitored piggery is monitored automatically according to the piggery monitoring model, thus determining whether the monitored piggery occurs an epidemic disease quickly and accurately; and meanwhile, the method can be applied to monitoring the epidemic disease throughout a country, so that before the epidemic disease breaks out, the epidemic disease can be detected timely and a reasonable countermeasure can be made.

### BRIEF DESCRIPTION OF THE DRAWINGS

To describe the technical solutions in the embodiments of the present invention or in the prior art more clearly, the following briefly introduces the accompanying drawings required for describing the embodiments. Apparently, the accompanying drawings in the following description show merely some embodiments of the present invention, and a person of ordinary skill in the art may still derive other drawings from these accompanying drawings without creative efforts.
FIG. 1 is a flow chart of a method for monitoring an epidemic disease in a piggery provided by the present invention.
FIG. 2 is a structural diagram of a system for monitoring an epidemic disease in a piggery provided by the present invention.

### DETAILED DESCRIPTION

The following clearly and completely describes the technical solutions in the embodiments of the present invention with reference to the accompanying drawings in the embodiments of the present invention. Apparently, the described embodiments are merely a part rather than all of the embodiments of the present invention. All other embodiments obtained by a person of ordinary skill in the art based on the embodiments of the present invention without creative efforts shall fall within the protection scope of the present invention.

An objective of the present invention is to provide a method for monitoring an epidemic disease in a piggery and system, which can improve the efficiency of monitoring an epidemic disease in a piggery.

To make the foregoing objective, features, and advantages of the present invention clearer and more comprehensible, the present invention is further described in detail below with reference to the accompanying drawings and specific embodiments.

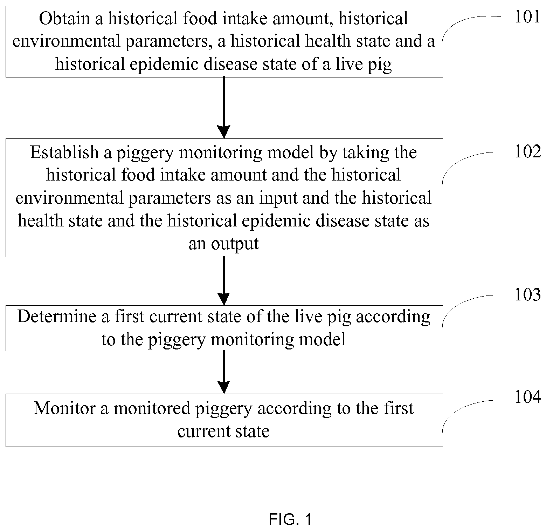

FIG. 1 is a flow chart of a method for monitoring an epidemic disease in a piggery provided by the present invention. As shown in FIG. 1, the method for monitoring an epidemic disease in a piggery includes the following steps.

Step 101: obtain a historical food intake amount, historical environmental parameters, a historical health state and a historical epidemic disease state of a live pig, where the historical environmental parameters include a temperature, a humidity, and concentration of gas in the air.

Step 102: establish a piggery monitoring model by taking the historical food intake amount and the historical environmental parameters as an input and the historical health state and the historical epidemic disease state as an output.

Step 103: determine a first current state of the live pig according to the piggery monitoring model, where the first current state includes a healthy state and an epidemic disease state.

The step 103 specifically includes: obtain a current food intake amount of the live pig within a period of time threshold range; determine whether the current food intake amount is in a downtrend within the period of time threshold range, and if the current food intake amount is in the downtrend, obtain current environmental parameters of the monitored piggery; determine whether the current environmental parameters and the historical environmental parameters are within an environmental-parameter similarity threshold range, and if yes, determine, by taking the current food intake amount and the current environmental parameters as an input, the first current state of the live pig according to the piggery monitoring model.

While a food intake amount of a group declines continuously, each environmental parameter in a piggery is normal, so it may be deduced that pigs are about to occur a disease; and after the group occurs the disease, symptoms from prediction of disease occurrence to the disease occurrence are recorded, e.g., the food intake amount declines quickly, the phenomenon that pigs die occurs continuously, etc.

When it is determined that the disease is not caused by a temperature, a humidity and an environmental problem, a piggery monitoring model under this environment is recorded and sorted out. When it is monitored that other piggeries also have the similar process, an analysis and a record may be made. When it is found that multiple piggeries within a range break out an epidemic disease condition suitable for this model in a same phase nearly, a nationwide early warning prompt is sent out in a platform system; and meanwhile, each piggery sends out an early warning prompt, so that a farmer makes preventive preparations in advance.

Step 104: monitor a monitored piggery according to the first current state.

After the step 104, the method further includes: obtain a second current state of any live pig in the monitored piggery; determine whether the second current state and the first current state are within a first current-state similarity threshold range; if yes, determine that no epidemic disease occurs in the monitored piggery; and if no, determine that an epidemic disease occurs in the monitored piggery and make an alarm to a farmer.

In response to determining that an epidemic disease occurs in the monitored piggery and making an alarm to a farmer, the method for monitoring an epidemic disease in a piggery further includes: obtain a third current state of a live pig in any piggery; determine whether the third current state and the first current state are within a second current-state similarity threshold range; if yes, determine that the epidemic disease does not occur in the any piggery; and if no, determine that the epidemic disease occurs in the any piggery and make an alarm to the farmer.

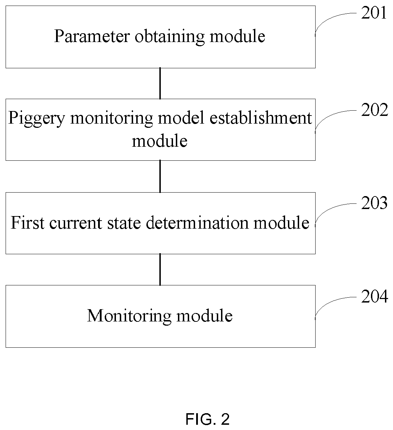

FIG. 2 is a structural diagram of a system for monitoring an epidemic disease in a piggery provided by the present invention. As shown in FIG. 2, the system for monitoring an epidemic disease in a piggery includes: a parameter obtaining module 201, a piggery monitoring model establishment module 202, a first current state determination module 203, and a monitoring module 204.

The parameter obtaining module 201 is configured to obtain a historical food intake amount, historical environmental parameters, a historical health state and a historical epidemic disease state of a live pig, where the historical environmental parameters include a temperature, a humidity, and concentration of gas in the air.

The piggery monitoring model establishment module 202 is configured to establish a piggery monitoring model by taking the historical food intake amount and the historical environmental parameters as an input and the historical health state and the historical epidemic disease state as an output.

The first current state determination module 203 is configured to determine a first current state of the live pig according to the piggery monitoring model, where the first current state includes a healthy state and an epidemic disease state.

The first current state determination module 203 specifically includes: a current food intake amount obtaining unit, configured to obtain a current food intake amount of the live pig within a period of time threshold range; a first judgment unit, configured to determine whether the current food intake amount is in a downtrend within the time threshold range to obtain a first determining result; a current environmental parameter obtaining unit, configured to obtain, if the first determining result indicates that the current food intake amount is in the downtrend within the time threshold range, current environmental parameters of the monitored piggery; a second judgment unit, configured to determine whether the current environmental parameters and the historical environmental parameters are within an environmental-parameter similarity threshold range to obtain a second determining result; and a first current state determination unit, configured to determine, if the second determining result indicates that the current environmental parameters and the historical environmental parameters are within the environmental-parameter similarity threshold range, by taking the current food intake amount and the current environmental parameters as an input, the first current state of the live pig according to the piggery monitoring model.

The monitoring module 204 is configured to monitor a monitored piggery according to the first current state.

The system for monitoring an epidemic disease in a piggery further includes: a second current state obtaining module, configured to obtain a second current state of any live pig in the monitored piggery; a third determining module, configured to determine whether the second current state and the first current state are within a first current-state similarity threshold range to obtain a third determining result; a monitored piggery monitoring module, configured to determine, if the third determining result indicates that the second current state and the first current state are within the first current-state similarity threshold range, that no epidemic disease occurs in the monitored piggery; and a first alarm module, configured to determine, if the third determining result indicates that the second current state and the first current state are not within the first current-state similarity threshold range, that an epidemic disease occurs in the monitored piggery and make an alarm to a farmer.

A third current state obtaining module is configured to obtain a third current state of a live pig in any piggery; a fourth determining module is configured to determine whether the third current state and the first current state are within a second current-state similarity threshold range to obtain a fourth determining result; an any piggery monitoring module is configured to determine, if the third current state and the first current state are within the second current-state similarity threshold range, that the epidemic disease does not occur in the any piggery; and a second alarm module is configured to determine, if the third current state and the first current state are not within the second current-state similarity threshold range, that the epidemic disease occurs in the any piggery and make an alarm to the farmer.

Each embodiment of the present specification is described in a progressive manner, each embodiment focuses on the difference from other embodiments, and the same and similar parts between the embodiments may refer to each other. For a system disclosed in the embodiments, since it corresponds to the method disclosed in the embodiments, the description is relatively simple, and reference can be made to the method description.

Several examples are used for illustration of the principles and implementation methods of the present invention.

## Claims

1. A method for monitoring an epidemic disease in a piggery, comprising obtaining, using a parameter obtaining module (201),
(101) a historical food intake amount, historical environmental parameters, a historical health state and a historical epidemic disease state of a live pig, wherein the historical environmental parameters comprise a temperature, a humidity, and concentration of gas in the air;
establishing (102) using a piggery monitoring model establishment module (202), a piggery monitoring model by taking the historical food intake amount and the historical environmental parameters as an input and the historical health state and the historical epidemic disease state as an output;
determining (103) by means of a first current state determination module (203), a first current state of the live pig according to the piggery monitoring model, wherein the first current state comprises a healthy state and an epidemic disease state; and
monitoring (104), using a monitoring module (204), a monitored piggery according to the first current state
wherein the determining (103) a first current state of the live pig according to the piggery monitoring model specifically comprises:
obtaining a current food intake amount of the live pig within a period of time threshold range;
determining whether the current food intake amount is in a downtrend within the time threshold range to obtain a first determining result;
if the first determining result indicates that the current food intake amount is in the downtrend within the time threshold range, obtaining current environmental parameters of the monitored piggery;
determining whether the current environmental parameters and the historical environmental parameters are within an environmental-parameter similarity threshold range to obtain a second determining result; and
if the second determining result indicates that the current environmental parameters and the historical environmental parameters are within the environmental-parameter similarity threshold range, determining, by taking the current food intake amount and the current environmental parameters as an input, the first current state of the live pig according to the piggery monitoring model.

2. The method for monitoring an epidemic disease in a piggery according to claim 1, after monitoring (104) the monitored piggery according to the first current state, further comprising:
obtaining a second current state of any live pig in the monitored piggery;
determining whether the second current state and the first current state are within a first current-state similarity threshold range to obtain a third determining result;
if the third determining result indicates that the second current state and the first current state are within the first current-state similarity threshold range, determining that no epidemic disease occurs in the monitored piggery; and
if the third determining result indicates that the second current state and the first current state are not within the first current-state similarity threshold range, determining that an epidemic disease occurs in the monitored piggery and making an alarm to a farmer.

3. The method for monitoring an epidemic disease in a piggery according to claim 2, after determining that the epidemic disease occurs in the monitored piggery and making the alarm to the farmer, further comprising:
obtaining a third current state of a live pig in any piggery;
determining whether the third current state and the first current state are within a second current-state similarity threshold range to obtain a fourth determining result;
if the third current state and the first current state are within the second current-state similarity threshold range, determining that the epidemic disease does not occur in the any piggery; and
if the third current state and the first current state are not within the second current-state similarity threshold range, determining that the epidemic disease occurs in the any piggery and making an alarm to the farmer.

4. A system for monitoring an epidemic disease in a piggery, comprising
a parameter obtaining module (201), configured to obtain a historical food intake amount, historical environmental parameters, a historical health state and a historical epidemic disease state of a live pig, where the historical environmental parameters comprise a temperature, a humidity, and concentration of gas in the air;
a piggery monitoring model establishment module (202), configured to establish a piggery monitoring model by taking the historical food intake amount and the historical environmental parameters as an input and the historical health state and the historical epidemic disease state as an output;
a first current state determination module (203), configured to determine a first current state of the live pig according to the piggery monitoring model, where the first current state comprises a healthy state and an epidemic disease state; and
a monitoring module (204), configured to monitor a monitored piggery according to the first current state;
wherein the first current state determination module (203) specifically comprises:
a current food intake amount obtaining unit, configured to obtain a current food intake amount of the live pig within a period of time threshold range;
a first judgment unit, configured to determine whether the current food intake amount is in a downtrend within the time threshold range to obtain a first determining result;
a current environmental parameter obtaining unit, configured to obtain, if the first determining result indicates that the current food intake amount is in the downtrend within the time threshold range, current environmental parameters of the monitored piggery;
a second judgment unit, configured to determine whether the current environmental parameters and the historical environmental parameters are within an environmental-parameter similarity threshold range to obtain a second determining result; and
a first current state determination unit, configured to determine, if the second determining result indicates that the current environmental parameters and the historical environmental parameters are within the environmental-parameter similarity threshold range, by taking the current food intake amount and the current environmental parameters as an input, the first current state of the live pig according to the piggery monitoring model.

5. The system for monitoring an epidemic disease in a piggery according to claim 4, further comprising:
a second current state obtaining module, configured to obtain a second current state of any live pig in the monitored piggery;
a third determining module, configured to determine whether the second current state and the first current state are within a first current-state similarity threshold range to obtain a third determining result;
a monitored piggery monitoring module, configured to determine, if the third determining result indicates that the second current state and the first current state are within the first current-state similarity threshold range, that no epidemic disease occurs in the monitored piggery; and
a first alarm module, configured to determine, if the third determining result indicates that the second current state and the first current state are not within the first current-state similarity threshold range, that an epidemic disease occurs in the monitored piggery and make an alarm to a farmer.

6. The system for monitoring an epidemic disease in a piggery according to claim 5, further comprising:
a third current state obtaining module, configured to obtain a third current state of a live pig in any piggery;
a fourth determining module, configured to determine whether the third current state and the first current state are within a second current-state similarity threshold range to obtain a fourth determining result;
an any piggery monitoring module, configured to determine, if the third current state and the first current state are within the second current-state similarity threshold range, that the epidemic disease does not occur in the any piggery; and
a second alarm module, configured to determine, if the third current state and the first current state are not within the second current-state similarity threshold range, that the epidemic disease occurs in the any piggery and make an alarm to the farmer.

## Patentansprüche

1. Verfahren zur Überwachung einer epidemischen Krankheit in einer Schweinezucht, umfassend:
Erhalten, unter Verwendung eines Parametererfassungsmoduls (201), (101) einer historischen Futteraufnahmemenge, historischer Umgebungsparameter, eines historischen Gesundheitszustands und eines historischen Zustands einer epidemischen Krankheit eines lebenden Schweins, wobei die historischen Umweltparameter eine Temperatur, eine Feuchtigkeit und eine Konzentration von Gas in der Luft umfassen;
Erstellen (102), unter Verwendung eines Schweinezucht-Überwachungsmodell-Erstellungsmoduls (202), eines Schweinezucht-Überwachungsmodells, indem die historische Futteraufnahmemenge und die historischen Umgebungsparameter als eine Eingabe und der historische Gesundheitszustand und der historische Zustand einer epidemischen Krankheit als eine Ausgabe verwendet werden;
Feststellen (103), mittels eines ersten Feststellungsmoduls (203) eines aktuellen Zustands, eines ersten aktuellen Zustands des lebenden Schweins gemäß dem Schweinezucht-Überwachungsmodell, wobei der erste aktuelle Zustand einen gesunden Zustand und einen Zustand einer epidemischen Krankheit umfasst; und
Überwachen (104), unter Verwendung eines Überwachungsmoduls (204), einer überwachten Schweinezucht gemäß des ersten aktuellen Zustands;
wobei das Feststellen (103) eines ersten aktuellen Zustands des lebenden Schweins gemäß dem Schweinezucht-Überwachungsmodell insbesondere Folgendes umfasst:
Erhalten einer aktuellen Futteraufnahmemenge des lebenden Schweins innerhalb eines zeitlichen Grenzbereichs;
Feststellen, ob die aktuelle Futteraufnahmemenge sich in einem Abwärtstrend innerhalb des zeitlichen Grenzbereichs befindet, um ein erstes Feststellungsergebnis zu erhalten;
wenn das erste Feststellungsergebnis anzeigt, dass sich die aktuelle Futteraufnahmemenge innerhalb des zeitlichen Grenzbereichs im Abwärtstrend befindet, Erhalten aktueller Umgebungsparameter der überwachten Schweinezucht;
Feststellen, ob die aktuellen Umgebungsparameter und die historischen Umgebungsparameter innerhalb eines Ähnlichkeitsgrenzbereichs der Umgebungsparameter liegen, um ein zweites Feststellungsergebnis zu erhalten; und
wenn das zweite Feststellungsergebnis anzeigt, dass die aktuellen Umgebungsparameter und die historischen Umgebungsparameter innerhalb des Ähnlichkeitsgrenzbereichs der Umgebungsparameter liegen, Feststellen des ersten aktuellen Zustands des lebenden Schweins gemäß dem Schweinezucht-Überwachungsmodells, indem die aktuelle Futteraufnahmemenge und die aktuellen Umgebungsparameter als eine Eingabe verwendet werden.

2. Verfahren zur Überwachung einer epidemischen Krankheit in einer Schweinezucht nach Anspruch 1, das nach dem Überwachen (104) der überwachten Schweinezucht gemäß dem ersten aktuellen Zustand ferner Folgendes umfasst:
Erhalten eines zweiten aktuellen Zustands eines beliebigen lebenden Schweins in der überwachten Schweinezucht;
Feststellen, ob der zweite aktuelle Zustand und der erste aktuelle Zustand innerhalb eines ersten Grenzbereichs für die Ähnlichkeit des aktuellen Zustands liegen, um ein drittes Feststellungsergebnis zu erhalten;
wenn das dritte Feststellungsergebnis anzeigt, dass der zweite aktuelle Zustand und der erste aktuelle Zustand innerhalb des ersten Grenzbereichs für die Ähnlichkeit des aktuellen Zustands liegen, Feststellen, dass keine epidemische Krankheit in der überwachten Schweinezucht auftritt; und
wenn das dritte Feststellungsergebnis anzeigt, dass der zweite aktuelle Zustand und der erste aktuelle Zustand nicht innerhalb des ersten Grenzbereichs für die Ähnlichkeit des aktuellen Zustands liegen, Feststellen, dass eine epidemische Krankheit in der überwachten Schweinezucht auftritt, und Abgeben eines Alarms an den Landwirt.

3. Verfahren zur Überwachung einer epidemischen Krankheit in einer Schweinezucht nach Anspruch 2, das nach dem Feststellen, dass die epidemische Krankheit in der überwachten Schweinezucht auftritt, und dem Abgeben eines Alarms an den Landwirt ferner Folgendes umfasst:
Erhalten eines dritten aktuellen Zustands eines lebenden Schweins in einer Schweinezucht;
Feststellen, ob der dritte aktuelle Zustand und der erste aktuelle Zustand innerhalb eines zweiten Grenzbereichs für die Ähnlichkeit des aktuellen Zustands liegen, um ein viertes Feststellungsergebnis zu erhalten;
wenn der dritte aktuelle Zustand und der erste aktuelle Zustand innerhalb des zweiten Grenzbereichs für die Ähnlichkeit des aktuellen Zustands liegen, Feststellen, dass die epidemische Krankheit in der Schweinezucht nicht auftritt; und
wenn der dritte aktuelle Zustand und der erste aktuelle Zustand nicht innerhalb des zweiten Grenzbereichs für die Ähnlichkeit des aktuellen Zustands liegen, Feststellen, dass die epidemische Krankheit in der beliebigen Schweinezucht auftritt, und Abgeben eines Alarms an den Landwirt.

4. System zur Überwachung einer epidemischen Krankheit in einer Schweinezucht, umfassend:
ein Parametererfassungsmodul (201), das konfiguriert ist, um eine historische Futteraufnahmemenge, historische Umgebungsparameter, einen historischen Gesundheitszustand und einen historischen Zustand einer epidemischen Krankheit eines lebenden Schweins zu erhalten, wobei die historischen Umgebungsparameter eine Temperatur, eine Feuchtigkeit und eine Konzentration von Gas in der Luft umfassen;
ein Schweinezucht-Überwachungsmodell-Erstellungsmodul (202), das so konfiguriert ist, dass es ein Schweinezucht-Überwachungsmodell erstellt, indem es die historische Futteraufnahmemenge und die historischen Umgebungsparameter als eine Eingabe und den historischen Gesundheitszustand und den historische Zustand einer epidemischen Krankheit als eine Ausgabe verwendet;
ein erstes Feststellungsmodul (203) eines aktuellen Zustands, das so konfiguriert ist, dass es einen ersten aktuellen Zustand des lebenden Schweins gemäß dem Schweinezucht-Überwachungsmodell feststellt, wobei der erste aktuelle Zustand einen gesunden Zustand und einen Zustand einer epidemischen Krankheit umfasst; und
ein Überwachungsmodul (204), das so konfiguriert ist, dass es eine überwachte Schweinezucht entsprechend dem ersten aktuellen Zustand überwacht;
wobei das erste Feststellungsmodul (203) eines aktuellen Zustands insbesondere Folgendes umfasst:
eine Einheit zur Ermittlung der aktuellen Futteraufnahmemenge, die konfiguriert ist, um eine aktuelle Futteraufnahmemenge des lebenden Schweins innerhalb eines zeitlichen Grenzbereichs zu ermitteln;
eine erste Beurteilungseinheit, die konfiguriert ist, um festzustellen, ob sich die aktuelle Futteraufnahmemenge innerhalb des zeitlichen Grenzbereichs in einem Abwärtstrend befindet, um ein erstes Feststellungsergebnis zu erhalten;
eine Einheit zur Ermittlung aktueller Umgebungsparameter, die konfiguriert ist, um aktuelle Umgebungsparameter der überwachten Schweinezucht zu erhalten, wenn das erste Feststellungsergebnis anzeigt, dass sich die aktuelle Futteraufnahmemenge innerhalb des zeitlichen Grenzbereichs im Abwärtstrend befindet;
eine zweite Beurteilungseinheit, die konfiguriert ist, um festzustellen, ob die aktuellen Umgebungsparameter und die historischen Umgebungsparameter innerhalb eines Grenzbereichs für die Ähnlichkeit der Umgebungsparameter liegen, um ein zweites Feststellungsergebnis zu erhalten; und
eine erste Einheit zur Feststellung des aktuellen Zustands, die so konfiguriert ist, dass sie, wenn das zweite Feststellungsergebnis anzeigt, dass die aktuellen Umgebungsparameter und die historischen Umgebungsparameter innerhalb des Grenzbereichs für die Ähnlichkeit der Umgebungsparameter liegen, unter Verwendung der aktuellen Futteraufnahmemenge und der aktuellen Umgebungsparameter als Eingabe den ersten aktuellen Zustand des lebenden Schweins gemäß dem Schweinezucht-Überwachungsmodell feststellt.

5. System zur Überwachung einer epidemischen Krankheit in einer Schweinezucht nach Anspruch 4, ferner umfassend:
ein zweites Modul zum Erhalt des aktuellen Zustands, das konfiguriert ist, um einen zweiten aktuellen Zustand eines beliebigen lebenden Schweins in der überwachten Schweinezucht zu erhalten;
ein drittes Feststellungsmodul, das konfiguriert ist, um festzustellen, ob der zweite aktuelle Zustand und der erste aktuelle Zustand innerhalb eines ersten Grenzbereichs für die Ähnlichkeit des aktuellen Zustands liegen, um ein drittes Feststellungsergebnis zu erhalten;
ein Überwachungsmodul für die überwachte Schweinezucht, das konfiguriert ist, um festzustellen, dass keine epidemische Krankheit in der überwachten Schweinezucht auftritt, wenn das dritte Feststellungsergebnis anzeigt, dass der zweite aktuelle Zustand und der erste aktuelle Zustand innerhalb des ersten Grenzbereichs des aktuellen Zustands liegen; und
ein erstes Alarmmodul, das konfiguriert ist, um festzustellen, dass eine epidemische Krankheit in der überwachten Schweinezucht auftritt, und um einen Landwirt zu alarmieren, wenn das dritte Feststellungsergebnis anzeigt, dass der zweite aktuelle Zustand und der erste aktuelle Zustand nicht innerhalb des ersten Grenzbereichs für die Ähnlichkeit des aktuellen Zustands liegen.

6. System zur Überwachung einer epidemischen Krankheit in einer Schweinezucht nach Anspruch 5, ferner umfassend:
ein drittes Modul zum Erhalt des aktuellen Zustands, das konfiguriert ist, um einen dritten aktuellen Zustand eines lebenden Schweins in einer Schweinezucht zu erhalten;
ein viertes Feststellungsmodul, das konfiguriert ist, um festzustellen, ob der dritte aktuelle Zustand und der erste aktuelle Zustand innerhalb eines zweiten Grenzbereichs für die Ähnlichkeit des aktuellen Zustands liegen, um ein viertes Feststellungsergebnis zu erhalten;
ein Überwachungsmodul für eine beliebige Schweinezucht, das konfiguriert ist, um festzustellen, dass die epidemische Krankheit in der beliebigen Schweinezucht nicht auftritt, wenn der dritte aktuelle Zustand und der erste aktuelle Zustand innerhalb des zweiten Grenzbereichs für die Ähnlichkeit des aktuellen Zustands liegen; und
ein zweites Alarmmodul, das konfiguriert ist, um festzustellen, dass die epidemische Krankheit in der beliebigen Schweinezucht auftritt und um einen Alarm an den Landwirt abzugeben, wenn der dritte aktuelle Zustand und der erste aktuelle Zustand nicht innerhalb des zweiten Grenzbereichs für die Ähnlichkeit des aktuellen Zustands liegen.

## Revendications

1. Procédé de surveillance d'une maladie épidémique dans une porcherie, comprenant les étapes de :
obtenir (101), en utilisant un module d'obtention de paramètres (201), une quantité de prise alimentaire historique, de paramètres environnementaux historiques, un état de santé historique et un état de maladie épidémique historique d'un porc vivant, dans lequel les paramètres environnementaux historiques comprennent une température, une humidité et une concentration de gaz dans l'air ;
établir (102), en utilisant un module d'établissement de modèle de surveillance de porcherie (202), un modèle de surveillance de porcherie en prenant en tant qu'entrée la quantité de prise alimentaire historique et les paramètres environnementaux historiques et en tant que sortie l'état de santé historique et l'état de maladie épidémique historique ;
déterminer (103), au moyen d'un premier module de détermination d'état actuel (203), un premier état actuel du porc vivant selon le modèle de surveillance de porcherie, dans lequel le premier état actuel comprend un état de santé sain et un état de maladie épidémique ; et
surveiller (104), en utilisant un module de surveillance (204), une porcherie surveillée selon le premier état actuel ;
dans lequel la détermination (103) d'un premier état actuel du porc vivant selon le modèle de surveillance de porcherie comprend spécifiquement les étapes de :
obtenir une quantité de prise alimentaire actuelle du porc vivant dans une plage de seuil de période de temps ; et
déterminer si la quantité de prise alimentaire actuelle est dans une tendance à la baisse dans la plage de seuil de période de temps pour obtenir un premier résultat de détermination ;
si le premier résultat de détermination indique que la quantité de prise alimentaire actuelle dans la dans la plage de seuil de période de temps est dans une tendance à la baisse, obtenir les paramètres environnementaux actuels de la porcherie surveillée ;
déterminer si les paramètres environnementaux actuels et les paramètres environnementaux historiques se situent dans une plage de seuil de similarité de paramètres environnementaux pour obtenir un deuxième résultat de détermination ; et
si le deuxième résultat de détermination indique que les paramètres environnementaux actuels et les paramètres environnementaux historiques se situent dans la plage de seuil de similarité de paramètres environnementaux, déterminer, en prenant en tant qu'entrée la quantité de prise alimentaire actuelle et les paramètres environnementaux actuels, le premier état actuel du porc vivant selon le modèle de surveillance de la porcherie.

2. Procédé de surveillance d'une maladie épidémique dans une porcherie selon la revendication 1 comprenant en outre, après l'étape de surveiller (104) la porcherie surveillée selon le premier état actuel, les étapes de :
obtenir un deuxième état actuel d'un porc vivant quelconque dans la porcherie surveillée ;
déterminer si le deuxième état actuel et le premier état actuel se situent dans une plage de seuil de similarité de premier état actuel pour obtenir un troisième résultat de détermination ; et
si le troisième résultat de détermination indique que le deuxième état actuel et le premier état actuel se situent dans la plage de seuil de similarité de premier état actuel, déterminer qu'aucune maladie épidémique ne se produit dans la porcherie surveillée ; et
si le troisième résultat de la détermination indique que le deuxième état actuel et le premier état actuel ne sont pas dans la plage de seuil de similarité de premier état actuel, déterminer qu'une maladie épidémique se produit dans la porcherie surveillée et en déclenchant une alarme à destination d'un fermier.

3. Procédé de surveillance d'une maladie épidémique dans une porcherie selon la revendication 2 comprenant en outre, après avoir déterminé que la maladie épidémique se produit dans la porcherie surveillée et avoir déclenché l'alarme à destination du fermier, les étapes de :
obtenir un troisième état actuel d'un porc vivant dans une porcherie quelconque ;
déterminer si le troisième état actuel et le premier état actuel se situent dans une plage de seuil de similarité de deuxième état actuel pour obtenir un quatrième résultat de détermination ;
si le troisième état actuel et le premier état actuel se situent dans la plage de seuil de similarité de deuxième état actuel, déterminer que la maladie épidémique n'est pas présente dans la porcherie quelconque ; et
si le troisième état actuel et le premier état actuel ne se situent pas dans la plage de seuil de similarité de deuxième état actuel, déterminer que la maladie épidémique se produit dans la porcherie quelconque, et déclencher une alarme à destination du fermier.

4. Système de surveillance d'une maladie épidémique dans une porcherie, comprenant :
un module d'obtention de paramètres (201), configuré pour obtenir une quantité de prise alimentaire historique, des paramètres environnementaux historiques, un état de santé historique et un état de maladie épidémique historique d'un porc vivant, dans lequel les paramètres environnementaux historiques comprennent une température, un taux d'humidité, et une concentration de gaz dans l'air ;
un module d'établissement de modèle de surveillance de porcherie (202), configuré pour établir un modèle de surveillance de porcherie en prenant en tant qu'entrée la quantité de prise alimentaire historique et les paramètres environnementaux historiques et en tant que sortie l'état de santé historique et l'état de maladie épidémique historique ;
un module de détermination de premier état actuel (203), configuré pour déterminer un premier état actuel du porc vivant selon le modèle de surveillance de porcherie, dans lequel le premier état actuel comprend un état de santé sain et un état de maladie épidémique ; et
un module de surveillance (204), configuré pour surveiller une porcherie surveillée en fonction du premier état actuel ;
dans lequel le module de détermination de premier état actuel (203) comprend spécifiquement :
une unité d'obtention de quantité de prise alimentaire actuelle, configurée pour obtenir une quantité de prise alimentaire actuelle du porc vivant au cours d'une plage de seuil de période temps ;
une première unité de jugement, configurée pour déterminer si la quantité de prise alimentaire actuelle est dans une tendance à la baisse dans la plage de seuil de période de temps pour obtenir un premier résultat de détermination ;
une unité d'obtention de paramètres environnementaux actuels, configurée pour obtenir, si le premier résultat de détermination indique que la quantité de prise alimentaire actuelle est dans la tendance à la baisse dans la plage de seuil de période de temps, des paramètres environnementaux actuels de la porcherie surveillée ;
une deuxième unité de jugement, configurée pour déterminer si les paramètres environnementaux actuels et les paramètres environnementaux historiques se situent dans une plage de seuil de similarité de paramètres environnementaux afin d'obtenir un deuxième résultat de détermination ; et
une première unité de détermination de l'état actuel, configurée pour déterminer, si le deuxième résultat de détermination indique que les paramètres environnementaux actuels et les paramètres environnementaux historiques sont dans la plage de seuil de similarité de paramètres environnementaux, en prenant en tant qu'entrée la quantité de prise alimentaire actuelle et la quantité de prise alimentaire historique, le premier état actuel du porc vivant selon le modèle de surveillance de porcherie.

5. Système de surveillance d'une maladie épidémique dans une porcherie selon la revendication 4, comprenant en outre :
un module d'obtention de deuxième état actuel, configuré pour obtenir un deuxième état actuel d'un porc vivant quelconque dans la porcherie surveillée ;
un troisième module de détermination, configuré pour déterminer si le deuxième état actuel et le premier état actuel se situent dans une plage de seuil de similarité de premier état actuel pour obtenir un troisième résultat de détermination ;
un module de surveillance de porcherie surveillée, configuré pour, si le troisième résultat de détermination indique que le deuxième état actuel et le premier état actuel se situent dans la plage de seuil de similarité de premier état actuel, déterminer qu'aucune maladie épidémique ne se produit dans la porcherie surveillée ; et
un premier module d'alarme, configuré pour, si le troisième résultat de détermination indique que le deuxième état actuel et le premier état actuel ne sont pas dans la plage de seuil de similarité de premier état actuel, déterminer qu'une maladie épidémique se produit dans la porcherie surveillée et déclencher une alarme à destination d'un fermier.

6. Système de surveillance d'une maladie épidémique dans une porcherie selon la revendication 5, comprenant en outre :
un module d'obtention d'un troisième état actuel, configuré pour obtenir un troisième état actuel d'un porc vivant dans une porcherie quelconque ;
un quatrième module de détermination, configuré pour déterminer si le troisième état actuel et le premier état actuel se situent à l'intérieur dans une plage de seuil de similarité de second état actuel pour obtenir un quatrième résultat de détermination ; et
un module de surveillance de d'une porcherie quelconque, configuré pour, si le troisième état actuel et le premier état actuel se situent dans la plage de seuil de similarité de deuxième état actuel, que la maladie, déterminer que la maladie épidémique ne se produit pas dans la porcherie quelconque ; et
un deuxième module d'alarme, configuré pour, si le troisième état actuel et le premier état actuel ne sont pas dans la plage de seuil de similarité de deuxième état actuel, déterminer que la maladie épidémique se produit dans la porcherie quelconque et émettre une alarme à destination du fermier.
